# EUROPEAN PATENT SPECIFICATION

(11) **EP 1 220 676 B1**
(45) Date of publication and mention of the grant of the patent: **04.05.2005**
(21) Application number: 00962269.7
(22) Date of filing: 29.09.2000
(51) Int. Cl.: A61K 33/06, A61K 31/593, A61K 31/616, A61P 35/00

(54) **PREVENTION OF COLORECTAL CANCER**
VORBEUGUNG VON KOLOREKTALKREBS
PREVENTION DU CANCER COLORECTAL

(30) Priority: 29.09.1999 DK 139099
(43) Date of publication of application: 10.07.2002
(73) Proprietor: Colotech A/S, 1437 Copenhagen K (DK); Raskov, Hans Henrik, 1850 Frederiksberg C. (DK)
(72) Inventor: RASKOV, Hans, Henrik, DK-1850 Frederiksberg C (DK)
(74) Representative: Plougmann & Vingtoft A/S
(86) International application number: PCT/DK2000/000546
(87) International publication number: WO 2001/022974

(56) References cited:
- US-A- 5 770 215
- J.R. Coll.Surg. Edinb., Volume 42, June 1997, S.J. DWERRYHOUSE et al, "Non-cytotoxic control of colorectal cancer" pages 147 - 153, XP002901460
- Leukemia Research, Volume 22, No. 2, 1998, John A. Soloski et al, "INDUCTION OF THE DIFFERENTIATION OF HL-60 PROMYELOCYTIC LEUKEMIA CELLS BY NONSTREROIDAL ANTIINFLAMMATORY AGENTS IN COMBINATION WITH LOW LEVELS OF VITAMIN D3" pages 153 - 161, XP002901461
- Carcinogenesis, Volume 16, No. 4, 1995, Barbara C. Pence et al, "Chemopreventive effects of calcium but not aspirin supplementation in cholic acid-promoted colon carcinogenesis: correlation with intermediate endpoints" page 757 - 765, XP002901462

## Description

The present invention relates to the use of acetylsalicylic acid (ASA), 1,25 dihydroxycholecalciferol (1,25 DHC) and calcium together with a pharmaceutically acceptable carrier for the preparation of a medicament for preventing the initiation and/or progression of colorectal cancer in a human. The invention also relates to a pharmaceutical medicament, comprising a combination of ASA, 1,25 DHC and calcium in a combination dosage together with a pharmaceutically acceptable carrier.

Colorectal cancer (CRC) is one of the leading cancer forms in the western world (1.3 million per year and over 600,000 annual deaths). In Denmark, the incidence is approximately 65 pr. 100.000 inhabitants and correlates to age. Concurrently with a fall in tobacco smoking in western industrial countries and an increased life expectancy, CRC is expected to become the most frequent solid cancer over the next decades.

The great majority of CRC cases are sporadic cancers, for which it is not possible to establish a genetic disposition. Effective CRC prevention in well-defined risk groups would have a significant effect on population health.

In the average population the lifetime risk of getting CRC is 6 per cent, and the risk of dying from the disease is 3 per cent (1,2,3). In first-degree relatives of patients with CRC, the risk is several times higher. In rare cases, the CRC disposing factors are hereditary non-polyposis colorectal cancer (HNPCC), where it is possible to establish the presence of mutations in mismatch repair genes, familial adenomatous polyposis (FAP, mutation in the APC gene), or inflammatory bowel diseases (ulcerative colitis and Crohn's disease), these factors accounting for 5 to 15 per cent in all.

There is no doubt that foods are the most important causal factor, including animal proteins and fats, which the western world is increasingly eating in excess amounts instead of cereals, fruits and vegetables. The incidence of CRC is increasing, but it is only half the magnitude among vegetarians as among meat-eaters (4). The progress made during the last decades within surgical techniques, adjuvant treatment, etc, has not lowered mortality to any mentionable degree. CRC screening means tracing cancers at an early stage and removal of intestinal polyps, but so far, however, studies have not shown screening to reduce the incidence. The overall five-year survival in Denmark is approximately 30 per cent and depends on the stage at the time of diagnosis. Approximately 25 per cent of the patients have disseminated cancer at the time of diagnosis and are beyond a cure. Three fourths of CRC patients undergo surgery intended to cure; nevertheless, 50 per cent of these patients die within five years because of recurrence.

With the choices and results of treatment known today, only effective prophylaxis will be able to reduce decisively CRC morbidity and mortality (3,5).

In recent years, focus is very much on cancer prophylaxis, in acknowledgement of the fact that that surgery mostly does not suffice as the only modality and that most cytotoxic regimens are ineffective against solid tumours.

The term chemoprophylaxis covers the use of pharmacologically active, non-cytotoxic agents or naturally occurring nutrients that protect against the emergence and development of clones of mutated, malignant cells.

In 1994, to analyse existing data and to initiate new studies, the National Cancer Institute, USA, established a Chemopreventive Branch. The NCI-CB has concluded that CRC is an attractive target for cancer chemoprophylaxis, since it is a frequent cancer with a high mortality. However no acceptable treatment is available.

A well-defined multistage carcinogenesis has been mapped with well-defined precursors in the form of colorectal adenomas, and the groups at risk are also well defined.

Some studies have pointed to an inverted correlation between the individual intake of non-steroid anti-inflammatory drugs (NSAID), and on calcium and vitamin D₃ and the risk of developing CRC. The studies in question are animal experimental models of colorectal carcinogenesis, prospective studies of patients with FAP and epidemiological studies taking the form of retrospective case-control studies and prospective cohort and interventional studies (6-17). In conclusion, 21 of 23 epidemiological studies have shown that regular use of NSAIDs reduces the risk of CRC by up to 50 per cent (18). However, data are not clear regarding dosage and duration of use. The most frequently studied drugs are acetylsalicylic acid, sulindac, piroxicam and indomethacin.

A few review articles and editorials have been published which find the results interesting, but existing data have not led to any recommendations proper nor proved any clinical significance (12). This is primarily because of the well-known undesirable effects of NSAIDs and the acknowledgement that the strongest evidence of the effect of these agents does not exist, ie, prospective, randomised double-blind trials in human populations.

The American Cancer Society has concluded that current data from epidemiological, clinical, pharmacological and toxicological studies show that acetylsalicylic acid protects against CRC development (13), and the FDA is currently assessing whether acetylsalicylic acid taken alone should be approved as chemoprophylaxis of CRC, or whether further phase III studies will be necessary.

Animal experimental data draw a promising picture of pharmacologically active drugs that, with different mechanisms of action, appear to be effective chemoprophylactic agents. However, individual epidemiological studies of calcium and vitamin D (or milk products) in relation to CRC are inconsistent. Out of 13 studies of calcium (nine case-control and four cohort studies), eight show a significant inverse correlation, three studies report insignificant correlation, whereas two fail to show any correlation.

Out of five epidemiological studies (three cohort studies and two case-control studies) of the impact of vitamin D on the CRC risk, two show a significant inverse correlation, whereas the rest have no significance.

The sequence of epithelium - adenoma - carcinoma is a process taking many years (5 to 10 years). CRC differs from many other cancers in as much as mutations in cell-cycle regulating genes and geneproducts are rarely seen. CRC is characterised by mutations in critical tumor suppressor genes (APC, DCC, p53, MCC) and oncogenes (K-ras) and upregulation in growth factors (especially the EGF-family) and enzymatic activity (especially cyclooxygenase).

In spite of CRC being a frequent cancer form, its incidence is only 65 per 100,000 inhabitants, and a clinical controlled study with the endpoints being invasive cancer and cancer-related deaths would require enrolment of tens of thousands individuals, and it would run for several decades and require astronomical financial resources. Healthy individuals do not feel impelled to participate in scientific studies including long-term medicine intake, and the results of any such studies would be subject to confounding.

Over 95 per cent of CRC develop from adenomas, which are accepted CRC precursors in scientific studies of humans and in animal experiments. Other biomarkers include genomic changes (mutations, etc), aberrant crypt foci (ACF), ornithine decarboxylase activity, cyclooxygenase activity and the prostaglandin level in mucosa, which are used as intermediary endpoints.

### Colorectal carcinogenesis and Calcium:

In the western world, the daily average intake of calcium is substantially below the recommended dietary allowance (RDA) of 800 to 1200 mg/day, increasing to 1500 mg /day for the elderly. In western countries, each adult has an average daily intake of 750 to 850 mg of calcium (14).

Approximately 30 per cent of dietary calcium is absorbed from the intestinal canal, and vitamin D₃ stimulates the absorption. The absorption is both transcellular (at low dietary calcium content) and paracellular. The residual amount of calcium in the intestinal lumen binds free fatty acids and secondary bile acids by formation of insoluble soaps and reduces the local irritant effect of these acids in the colon.

Deoxycholic acid (DCA) in particular, which produces epitheliolysis in the epithelial surface of the colon, is considered to be the most carcinogenic and mitogenic of the secondary bile acids (15). Epitheliolysis induces potent proliferation in the crypts, probably as a result of exposure of the basal membrane. In the activated phase (S phase), the cells are more sensitive towards carcinogens like DCA, free fatty acids, etc (16). *In vivo* the harmful effect of 5mM of DCA can be prevented by increasing the Ca concentration in the intestinal lumen from 0 to 4 mM.

In several cases, the results of case-control studies and cohort studies have shown a significant correlation between a high dietary calcium level and a reduction in the risk of CRC development. However, the results are not unambiguous, although large volumes of data from animal experimental studies all point in the same direction (14). One prospective study shows a significant reduction in the rate of polyp recurrence and a significant increase in cancer-related survival following CRC surgery at calcium supplements (calcium carbonate 2 g/day (17).

The formation of insoluble calcium soaps is still considered to be the most important mechanism of the cancer preventive action of calcium, but in recent years focus has increasingly been directed at the central role of calcium in intracellular signal transduction, Calcium is a key factor in maintaining normal cell membrane function, and calcium flux over the cell membrane plays a central role in mediating intracellular signal transduction, which regulates multiple cellular functions. Furthermore, the expression of cellular surface cadherins, which is necessary to maintain intercellular contact, depends on the presence of calcium. Particularly on colon cancer cells the expression of cadherins correlates with the rate of differentiation and to the clinical outcome (19).

Reduction of calcium concentration in intercellular fluid lowers cell response to growth regulating factors and reduces the permeability of cell membranes. When the calcium concentration is reduced, the rates of proliferation and dedifferentiation increase.

Calcium contributes to regulating all cell division and cell differentiation phases, primarily through activation of various protein kinases (cAMP-dependent kinase, Ca-calmodulin-dependent protein kinases, protein kinase C) (20,21). Calcium suppresses omithine-decarboxylase, a tumour-promoting enzyme (14) and reduces the number of K-ras mutations in colonic epithelium stimulated with the carcinogen 1,2 dimethylhydrazine (22). K-ras mutations are one of the early genomic changes in the carcinogenesis. K-ras mutations occur in approximately 85 per cent of adenocarcinomas and approximately 55 per cent of adenomas, but ras mutations exist even in up to 50 per cent of ACF.

Elevated calcium values produce increased differentiation of epithelial cells with concurrent growth suppression, but neoplastic colonic epithelial cells presumably lose their calcium response at one of the late stages of the epithelium - carcinoma sequence (16).

Stimulation with carcinogens at the preneoplastic stage produces luminal proliferation of the colon crypt proliferating cells, an increased proliferation ratio and increased incidence of ACF, which is also seen in individuals with an elevated risk of colon cancer (HNPCC and FAP patients). At calcium administration, cells in the crypts can be converted to a normal proliferation ratio and normal geographical distribution of non-dividing cells in the luminal two thirds of the crypts and proliferating cells at the bottom of the crypts (23).

Montoya R.G. et all, "Chemoprevention of gastrointestinal cancer." CANCER AND METASTASIS REVIEWS (1997) 16/3-4 (405-419) disclose several compounds used for the prevention of colon cancer. There is no mentioning om vit D₃ and the reference to Ca relates to the theory concerning formation of insoluble calcium soaps.

WO 96 41645 disclose use of COX₂ inhibitors for use in the treatment of imfammation.

Pence B C et al: Experimental chemoprevention of colon carcinogenesis by combined calcium and aspirin (Meeting Abstract), Proc. Annu Meet AM Assoc Cancer Res (1994). Vol. 35, pp A3719. ISSN: 0197-016X describes that tumor burden was lowest in groups fed Ca or ASA during promotion only. Supplementation during progression was less effective.

Sokoloski, John A. et al: Introduction of the differentation of HL-160 romyelocytic leukemia cells by nonsteroidal anti-inflammatory agents in combination wiht low levels of Vit D₃; Leuk. Res (1998), 22(2), 153-161, 1998. This article disclose that D₃ has an increasing effect on NSAID, however only derivatives with receptor-binding properties has this effect and not D₃ analoges without receptorbinding effect and with the Ca increasing effect has this increasing effect on the NSAID. The stydy is performed with leukemia cells.

### Colorectal carcinogenese and Vitamin D₃:

Vitamin D₃ (D₃) increases serum calcium by furthering the absorption of calcium and phosphate from the intestinal canal and mobilising calcium from bones. D₃ is present in food; it is formed by ultraviolet radiation of 7-dehydrocholesterol, a provitamin present in human skin and in fatty tissues in many animals. D₃ metabolises by successive hydroxylation, first in the liver, into 25-hydroxycholecalcipherol, and then in the kidneys to 1,25 dihydroxycholecalcipherol (1,25DHC) or 24,25DHC, which are the hormonally active metabolites of D₃ (1,25DHC > 24,25DHC).

In addition to its anti-oxidative effect, 1,25DHC resembles steroid hormones in its chemical structure and mechanism of action, as 1,25DHC passes the cell membrane and binds to a specific cytoplasmatic receptor protein. This hormone-receptor complex is activated during translocation into the cell nucleus where it binds to DNA and initiates mRNA transcription and protein synthesis. In the nuclear membrane receptors for 1,25DHC are situated (high affinity nuclear vitamin D receptors, VDR), which contribute to regulating the calcium flux over cell membranes (17).

1,25DHC modulates signal transduction, inhibits proliferation and DNA synthesis, modulates c-myc, c-fos and c-jun oncogenic expression, induces differentiation and presumably apoptosis. VDRs have been identified both in normal colonic mucosa and in colorectal carcinomas (24). 1,25DHC increases intracellular calcium and stimulates various protein kinases. 1,25DHC stimulates transcription of the calbindin D gene in colonocytes, which is believed to increase transcellular calcium absorption.

A potent upregulation (300-400 per cent) of VDR takes place in neoplastic colonocytes; this can be interpreted as an adaptive response to tumoric cell growth, by which the cell increases its differentiation potential. This response disappears at more advanced stages of the disease (>T3), where it is assumed that the vitamin D defence mechanism becomes inactivated (25).

*In vitro,* 1,25DHC inhibits the growth of human colon cancer cell lines (LoVo) including CEA-producing cell lines. *In vivo* (mice), 1,25DHC can suppress growth of solid, human xenografts (17,26). A few cohort studies of human populations have shown a significant reduction in the risk of developing colorectal cancer at intake of vitamin D₃ (or its active metabolite 1,25DHC), resulting in serum concentrations over 20 ng/ml (6,7).

RDA for vitamin D₃ is 10 µg/day increasing to 20 µg/day in elderly women without oestrogen substitution (6,7,27). The recommended dose of 1,25DHC is 0.01 µg/kg BW three times a week. In osteoporosis studies, 0,75 µg/day has been shown to induce hypercalcaemia.

New synthetic D₃ analogue preparations have 100-200 times the antiproliferative effect and effect on differentiation and only 0.5 times the hypercalcaemic effect of 1,25DHC.

Two NCI-CB sponsored studies of 1,25DHC 0.5 µg or D3 400 IU and calcium carbonate 1500 mg have been initiated in 1994.
It is known from studies of bone mineral turnover that vitamin D and calcium are mutually dependent factors, and this has proved to be the case also in the regulation of cell division and cell differentiation.

Colorectal carcinogenesis and cyclooxygenase inhibitors (acetylsalicylic acid (ASA) and other NSAIDs) and CRC:

The regulating effect of cyclooxygenase inhibitors (COX inhibitors) on the colonic epithelium has been investigated in connection with treatment of chronic inflammatory intestinal diseases and FAP.

A cancer preventive effect of COX inhibitors has not been identified in details at the level of molecular biology, but it is considered to be related to the impact of these drugs on arachidonic acid metabolism and prostaglandin synthesis via blocking of cyclooxygenase enzymes (COX).

Two isomeric forms have been identified: COX₁ and COX₂:

COX, is the constitutive form. In the upper gastrointestinal tract it affects the protection of mucosa by inducing bicarbonate secretion and mucin production primarily through prostaglandin E (PGE), which is the quantitatively dominant product of the COX₁ turnover of arachidonic acid. COX₂ is an inducible form. It is particularly induced by inflammatory stimuli, and it catalyses the formation of proinflammatory cytokines, including PGE₂ and PGF_{α}, which strengthen the mutagenic effect of carcinogens by proliferation induction, suppression of the immune system and stimulation of angiogenesis. PGE₂ exerts its inhibitory effect via negative feedback T-cell proliferation and lymfokine production.

Arachidonic acid (AA, 5,8,11,14-eicosatetraenoic acid) originates from the cell turnover of phospholipids (PL) located in the cell membrane. AA is primarily liberated from PL by hydrolysis of the ester binding that binds AA to PL. In most cell types this occurs by direct activation of the enzyme phospholipase A2. The phospholipase A2 activity constitutes the common factor regulating the rate of AA liberation, and thus the rate of production for all eicosanoids (PG, prostacyclins, thromboxanes and leukotrienes).

AA metabolises via the COX pathway to eicosanoids, which stimulate cell division, as it is seen in inflammatory conditions, or via the lipoxygenase pathway to hydroperoxides (HPETE) and hydroxy compounds (HETE). The third pathway for arachidonic acid metabolism is via cytochrome P450 to HETE and EET (epoxyeicosatrienic acid). It has been shown that blocking of the lipoxygenase activity inhibits growth-factor-induced colonic tumor cell proliferation (28).

The COX inhibitor ASA (aspirin and others) and its metabolite salicylate block the formation of PG from AA by irreversible acetylation of COX (29), denying AA access to the active part of the enzyme. The COX activity can only be re-established by production of new COX molecules, and therefore cells without protein synthesis, such as platelets, are not capable of resuming COX activity. The main chemopreventive effect of ASA is deemed to be COX₂ inhibition (30), which results in metabolising of AA via a lipoxygenase pathway to 15-HETE (leukotriene with anti-inflammatory and antimitogenic effects).

Most other NSAIDs (piroxicam, sulindac and indomethacin) block COX in a reversible and dose-dependent manner, wherefore ASA is a more potent PG inhibitor. As it appears from the above, there are several mechanisms of action, and the PG cascade also depends on the calcium-regulated signal transduction system (21).

Several classic carcinogens are used as electron donors during the COX reaction, and they are activated by this reaction (high DNA affinity). Among them are polycyclic aromatic hydrocarbons, aflatoxins, halogenated pesticides, aromatic amines and phenol compounds. Thus, COX activates potential carcinogens into active DNA harmful metabolites.

*In vitro* studies show that most NSAIDs have an antiproliferative effect on human colon cancer cell lines (Ht-29, SW-80, DLD-1)(31).

*In vitro* studies also show that although the NSAID effect on the PG-synthesis is eliminated (for instance by the use of sulindac metabolites without COX inhibition), the growth of human colon cancer cell lines is inhibited nevertheless. This points to several mechanisms of action, including the ability significantly to induce apoptosis (28,32), and modulation of transmembrane calcium flux and intercellular junctions (33).

NSAID has been shown to inhibit several endonucleases; these are enzymes that cleave DNA molecules. Presumably they play a central part in the genomic instability that is one of the characteristics of colorectal multistep carcinogenesis (32). Other molecular biology mechanisms are discussed in detail in (35).

An interesting point is that, contrary to other NSAIDs, ASA has been shown to inhibit proliferation and lumen formation in cocultivated normal colon epithelial cells (carcinoma cells in compartment 2). This is taken as an expression of inhibition of the growth promoting signals of carcinoma cells. Other areas in which ASA differs from other NSAIDs are irreversible COX inhibition, lower plasma binding (approximately 50 per cent as compared with approximately 90 per cent).

Human colorectal neoplasms, both adenomas and adenocarcinomas, have been found to produce large amounts of PG, especially of the E type (31,36), and precisely COX₂ activity has been found to be accentuated 2 to 50-fold in 85 to 90 per cent of colorectal carcinomas (35). Particularly APC loss of heterozygocity (LOH) is believed to stimulate COX₂ expression at an early stage of neoplastic development in both epithelial and stroma cells. However, it may precisely be the stromal COX₁ activity that stimulates the expression of various angiogenetic factors (VEGF, bFGF and TGF_{β1}).

Other proneoplastic effects of COX are the change of TGF-beta from an anti-proliferative growth factor to an pro-proliferative growth factor and reduced intercellular and cellular-stromal contact/communication and thereby promoting angiogenesis and metastasis. These properties of COX suggests that inhibitions of both isoforms may have important effects against CRC (38).

In itself, one of the three domains of the COX molecule (COX domain, EGF-domain and membrane binding motif) resembles the epidermal growth factor (ligand for the EGF receptor is also TGF_{α}). For this reason a possible inactivation of the entire domaine would be interesting in atempt to achieve optimum prophylaxis.

The COX inhibitor ASA (aspirin and others) and its metabolite salicylate block the formation of PG from AA by irreversible acetylation of COX (37), denying AA access to the active part of the enzyme. The COX activity can only be re-established by production of new COX molecules, and therefore cells without protein synthesis, such as platelets, are not capable of resuming COX activity. The main chemopreventive effect of ASA is deemed to be COX₂ inhibition (38), which results in metabolising of AA via a lipoxygenase pathway to 15-HETE (leukotriene with anti-inflammatory and antimitogenic effects).

Most other NSAIDs (piroxicam, sulindac and indomethacin) block COX in a reversible and dose-dependent manner, wherefore ASA is a more potent PG inhibitor. As it appears from the above, there are several mechanisms of action, and the PG cascade also depends on the calcium-regulated signal transduction system (21).

Several major epidemiological studies of COX inhibitors in the form of cohort studies, case-control studies and prospective interventional studies have shown a significant preventive effect (reduction of relative risk of 40 to 50 per cent) particularly of ASA on CRC after long-term (2 to 10 years) therapy in the doses used to prevent ischaemic heart disease (11-13,39-42). In a cohort of patients with ulcerative colitis, a relative risk reduction of 0.38 (0,2 to 0,7) has been found following only 3 months of sulphasalazine therapy.

Animal trials have been able to demonstrate a significant protective effect (50 to 60 per cent) of for instance indomethacin and piroxicam in rats exposed to the carcinogen dimethylnitrosamine or azoxymethane (methylazoxymethanol)(43-46).

The most frequent undesirable effects connected with long-term administration of NSAIDs are gastroduodenal ulceration and bleeding because of low PG and thromboxane A₂ levels in the gastrointestinal tract. PG stimulates mucin production and bicarbonate secretion, and thromboxane A₂ indicates platelet aggregation. These complications are primarily related to inhibition of the constitutive COX₁ enzyme.

Undesirable effects and complications primarily relate to the use of NSAIDs as analgesic or anti-inflammatory agents in significantly higher doses, but they are potential sequels after long-term use also in lower doses.

A review of 16 cohort studies and case-control studies showed that the risk of developing severe NSAID-induced gastrointestinal undesirable effects amounts to 2 to 4 per cent a year at analgesic and anti-inflammatory daily doses (14). In low-dose aspirin prophylaxis of cardiovascular disease the relative risk-reduction in relation to stroke, acute myocardial infarction and/or cardiovascular death was found to be approx. 25% (47).

The Physicians' Health Study (325 mg of acetylsalicylic acid qod) found that, in addition to a significant reduction of the risk of acute myocardial infarct, there were significantly more cases of melaena and epistaxis than in the placebo group, but of neither cerebral haemorrhage nor unspecific gastrointestinal bleeding (including haematemesis)(39).

There exist numerous data on the pharmacokinetics and toxicity of COX inhibitors, especially regarding ASA. The FDA has found that for instance acetylsalicylic acid is a safe and efficient anti-inflammatory and analgesic agent and well suited for over-the-counter sales. No further toxicological studies are necessary to assess the usage of acetylsalicylic acid in chemoprevention (48-49).

Dwerryhouse et al. (53) discloses the positive influence of the intake of calcium/vitamin D₃ on CRC. 1, 25-dihydroxycholecalciferol is identified as the active metabolite of vitamin D₃. This document also mentions the beneficial effect of aspirin for preventing CRC.

Patent US-5770215 is directed to multivitamin compositions for the inhibition of vascular occlusions. It discloses compositions containing vitamins, a vascular occlusion inhibiting substance, preferably ASA, and minerals. The vitamins include vitamin D₃ and the minerals include calcium. The usefulness for treating and preventing a wide variety of diseases is mentioned, thereincluded also the reduction ot the danger of CRC.

Pence et al. (54) discloses test results from an azoxymethane-induced colon cancer model in rats. Calcium turned out to be a more effective chemopreventive agent than ASA in cholic acid promoted colon carcinogenesis. ASA lacked any major protective effect in this study.

The carcinogenesis in colorectal cancer involves a number of genetic changes and epige-netic factors such as increased expression of growth factors and suppression of growth inhibitors, which does not necessarily imply underlying mutations (but which for instance occurs at increased COX expression). Data from epidemiological studies and animal trials show that vitamin D₃ and calcium may be pharmacologically active when used as chemoprophylaxis of CRC. However, the effect is moderate. Some epidemiological studies in human populations indicate a reduction (40 to 50 per cent) of the relative cancer risk in populations using ASA continuously; there is not, however, consensus as to the dosage and duration of treatment. The risk-reduction in relation to CRC could be twice the risk-reduction of cardiovascular events.

### BRIEF DESCRIPTION OF THE INVENTION

No prospective, randomised, double-blind studies exist. Studies of cancer chemoprophylaxis are extremely expensive, since they perforce have to include a very high number of individuals and run for years, if the study endpoints are to be invasive cancer and cancer-related mortality. For these reasons there is an increasing tendency towards relying on epidemiological studies of intermediary endpoints (eg, polyps, ACF, etc), animal trials of genetically engineered or carcinogenically stimulated animal populations and biological models for examining different biomarkers (mutations, growth factors, etc).

According to the present invention, the CRC preventive effect of the following combination of preparations manifests itself by a significant reduction of the incidence and overall morbidity and mortality of colorectal cancer. To achieve this effect, however, it is believed to be important to take the preparation consistently as prophylaxis over a long time (probably more than one year), exactly as for the prevention of ischaemic heart disease and osteoporosis.

By combining ASA with 1,25 DHC with calcium, a synergistic effect is achieved, so that the amounts of the individual drugs presumably are reduced and the toxicity thereby reduced to a negligible level.

According to the present invention a surprising effect may be obtained by a combination dosage comprising individual drugs which exert their effects on specific areas of the carcinogenesis: modification of signal transduction and expression of oncogenes, reduction of carcinogenic impact on the colonic epithelium and intracellular and intercellular signal transduction, COX inhibition and probably apoptosis.

In a preferred embodiment the human to be treated is a patient which due to underlying disease or a genetic defect is in risk of developing colorectal cancer such as: HNPCC patients, polyp patients, patients with a history of CRC. In addition, individuals over 50 years, who are first-degree relatives of patients with colorectal cancer (risk of developing CRC 2 to 4 times increased (12 to 25 per cent).

For first-degree relatives of individuals with CRC diagnosed before the age of 50 years or for individuals with two first-degree relatives with CRC, the risk increases 4 to 6 times (24 to 36 per cent) regardless of age.

For carriers of HNPCC mutations, the risk of CRC is 75 per cent at the age of 65 years and the risk of metachronous cancer is 45 per cent ten years following resection of the primary tumour.

For patients with chronic inflammatory intestinal diseases (ulcerative colitis and Crohn's disease), the risk increases 4 to 25 times (lifetime risk 12 to 75 per cent in patients not treated with surgery after more than ten years of illness) depending on the dissemination and duration of the disease.

Accordingly, the use according to the present invention is in a preferred embodiment suitable wherein the human is selected from the group being in risk of development of colorectal cancer due to being a first-degree relative to a patient with colorectal cancer, and/or carries the gene(s) for hereditary non-polyposis colorectal cancer (HNPCC), and/or having familial adenomatous polyposis, colorectal adenomas and/or a inflammatory bowel disease such as ulcerative colitis or Crohns disease.

The preparations could be combined as follows:

| | |
|---|---|
| 500 | mg of Calcium (calcium carbonate 1250 mg) and/or |
| 0.5 | µg of 1,25DHC (or vitamin D₃ 400 IU or D₃ analogue, eg, 0.25 µg of calcitriol or 0,005 µg of calcitriol/kg BW) and |
| 75 | mg of ASA or analogue reversible or irreversible COX₂ inhibitor |

The main requirements for a preparation designed for chemoprophylaxis include: low price, high compliance and ultra-low toxicity; it is assumed that by adding 1,25DHC and calcium the amount of COX inhibitor (ASA) can be reduced, so that the ASA-related undesirable effects can be reduced to a negligible level without reducing its action. For acetylsalicylic acid, the undesirable effects following long-term use have made the FDA hesitate before approving acetylsalicylic acid as chemoprophylaxis of CRC.

Preparations with specific action in the colon, such as for instance 5-ASA, may turn out to be appropriate, possibly in combination with mucosa-protective agents.

ASA and other NSAIDs are approved for over-the-counter sales for analgesic and anti-inflammatory use. Similarly, combination preparations containing D₃ and calcium (for instance calcium carbonate 1250 mg = calcium 500 mg + D₃ 400 IU) are sold OTC for osteoporosis prophylaxis.

At first, *in vivo* studies of the effect of the above has been carried out in the form of animal experiments with the Institute for Toxicology of the Danish Veterinary and Food Administration (DVFA). The results of the studies is shown in Table 1.

### DETAILED DESCRIPTION OF THE INVENTION

In one embodiment the invention relates to the reduction of the effective dosage of ASA in a chemoprofylactive treatment of colorectal cancer in a human by co-administration with a non toxic dosage of 1,25 DHC and Ca in the form of a combination dosage. This is due to the fact that it has surprisingly been shown that the addition of the 1,25 DHC and calcium may decrease the necessary dosage of acetylsalisylic acid in order to decrease the formation of abberant crypt foci in a model rat both with respect to size and numbers. Accordingly, the invention also relates to the prevention of the initiation and/or progression of colorectal cancer in a human comprising administration to the human a combination dosage of ASA, 1,25 DHC and calcium.

By prevention and chemoprofylactive effect is meant prevention of colorectal cancer or the initiation and/or progression of colorectal cancer and or the effect of reducing the formation of conditions being pre-malignant of colorectal cancer.

According to the present invention it is believed that the administration of the combination dosage should be administered regularly with an average daily dosage of ASA in the range of 50 mg and 500 mg, preferably in the range of 25 to 400 mg more preferred of in the range of 50 to 300 mg, still more preferred in the range of 75 to 150 mg such as in the range of 75 to 100 mg.

The combination dosage further comprises the vitamin D₃ metabolite 1,25 dihydroxycholecalcipherol in the range of 0,1 microgram to 2 microgram. The calcium in the combination dosage may preferable be in the range of 200 mg to 3500 mg such as calcium is in the range of from 250 mg to 3000 mg, such as in the range of from 300 mg to 2500 mg, preferable in the range of 400 to 2000 mg, more preferred in the range of from 500 to 1000 mg, such as 750 mg.

As mentioned above, the treatment or prevention should be continued for a long period in order to give the best effect, however it is believed that a beneficial effect may be obtained after a treatment of at least 3 months. Accordingly the administration is preferably continued for at least 6 months, such as a least for 1 year, preferable for at least 2 years. However, persons in high risk may be treated according with the present invention for the rest of lives.

In an important aspect, the administration of the combination dosage results in the prevention of the initiation or progression of colorectal cancer exceeding the effect of the administration of any of the individual ingredients in the same daily dosage and in the same period.

In the use according to the invention the combination dosage has a preventive effect which is synergistic compared with the effect of the individual effective ingredients.

An additive effect according to he invention may be calculated as an effect of the sum of prevention by each of the substances ASA, the Vitamin D_{3,} and the Ca, respectively or by an effect of the sum of prevention by the selection of two of the substances ASA, the Vitamin D₃ and the Ca and the preventive effect by the remaining substance.

By synergistic effect according to the present invention is preferable meant an effect which is higher than the additive effect as disclosed above. A suitable reference for the calculation is disclosed in Example 1 disclosing a study of the formation of abberent crypt foci in induced rat colon.

The COX inhibitor may be any one acting on one or more of the mechanisms selected from reversible or irreversible acetylation of COX_{1,} reversible or irreversible acetylation of COX₂, inhibition of angiogenesis, inhibition of arachidonic acid metabolism, blocking of AA metabolism, inhibits stimulation of proliferation stimulating from Epidermal Growth Factor, and stimulation of apoptosis. The COX inhibitor is ASA. The vitamin D₃ is 1,25 dihydroxycholecalcipherol.

The ASA is preferably acting by one or more of the following mechanisms: inhibiting of cell proliferation; inhibiton of upregulation of pro-proliferative agents such as growth factors; modulation of signal transduction; and induction of apoptosis. Also inhibition of angiogenesis, inhibition of arachidonic acid metabolism may be the target for the ASA. In a further aspect, the cyclooxygenase inhibitor is acting by decreasing the formation of potential carcinogens into DNA harmful metabolites.

The vitamin 1,25 DHC thereof is preferable acting by one or more of the following mechanisms: inhibiting of cell proliferation; inhibition of DNA synthesis; modulation of signal transduction; induction of differentation; and induction of apoptosis.

The active mechanism of the calcium is according to the present invention preferable an effect on the expression of cellular surface cadherins and intra- and extracell signal transmission.

One very important aspect of the the present invention is the finding that the use may reduce the risk of developing colorectal cancer in the individual human receiving the treatment by at least 10% or more compared to the effect of obtained by any of the individual ingredients in the same dosage and in the same period of administration. The reduction may be at least 20% or more, and in certain circumstances e.g. for high risk patients even 30% or more. The effect may be measured as disclosed in the example by the number of aberrent crypt foci in AOM induced rats receiving administration of the combination dosage.

The combination dosage according to the invention is the combination dosage comprising ASA, 1,25 DHC and Ca in that these ingredients are all well known drugs.

In a still further embodiment the invention relates to the use of ASA, 1,25 DHC and calcium together with a pharmaceutically acceptable carrier for the preparation of a medicament for preventing the initiation and/or progression of colorectal cancer in a human. In a preferred embodiment the medicament is in the form of a combination dosage comprising the ASA, 1,25 DHC and the calcium.

The use may be in accordance with any of the uses described above and a still further embodiment, the invention relates any such pharmaceutical medicament. The pharmaceutical medicament may accordingly comprise a combination of a cyclooxygenase (COX) inhibitor, a vitamin D₃ including analogues and metabolites thereof and/or calcium. In a further aspect, the pharmaceutical medicament is such a medicament as any of the combination dosages administered according to any of the uses described above.

Accordingly, a further aspect of the invention relates to the use of a cyclooxygenase (COX) inhibitor, a vitamin D₃ including analogues and metabolites thereof and calcium together with a pharmaceutically acceptable carrier for the preparation of a medicament for preventing the initiation and/or progression of colorectal cancer in a human.

In a still further aspect the present invention relates to a pharmaceutical medicament comprising a combination of a cyclooxygenase (COX) inhibitor, a vitamin D₃ including analogues and metabolites thereof and calcium in a combination dosage together with a pharmaceutically acceptabel carrier.

The pharmaceutical medicament according to the invention is preferably a medicament wherein the combination dosage comprises the vitamin D₃ metabolite 1,25 dihydroxycholecalcipherol in the range of 0,1 µg to 2 µg such as in the range of 0,2µ to 1.5 µg, preferable in the range of from 0.3 to 1 µg, more preferred in the range of from 0.4 µg to 0,75 µg, such as 0.5 µg.

In a further embodiment, the pharmaceutical medicament is one wherein the combination dosage comprises calcium in the range of 200 mg to 3000 mg, such as in the range of from 300 mg to 2500 mg, preferable in the range of 400 to 2000 mg, more preferred in the range of from 500 to 1000 mg, such as 750 mg.

In a preferred embodiment the pharmaceutical medicament according to the invention is wherein the combination dosage comprises ASA in the range of 50 mg and 500 mg, preferably in the range of 25 to 400 mg more preferred of in the range of 50 to 300 mg, still more preferred in the range of 75 to 150 mg such as in the range of 75 to 100 mg, The most preferred medicament comprises 50 to 75 mg ASA, 500-1000 mg Ca, and 0.5 to 1 µg 25 dihydroxycholecalcipherol.

By a dosage according to the present invention is meant individual dosages for instance in a one packet or a physical entity of one or more of the ingredient. The combination dosage may also comprise different COX inhibitors as well as different Vit D₃ analogues and or metabolites.

Accordingly, in a preferred embodiment, the pharmaceutical is one pharmaceutical comprising all three ingredients in order to secure the right individual dosage and patient compliance.

The COX inhibitors according to the invention in addition to the ASA include other NSAIDS known in the art.

### EXAMPLE 1

The influence of 1α,25 (OH)₂-vitamin D₃, calcium and acetylsalicylic acid on AOM-induced aberrant crypt foci and colorectal tumors in rat colon

### Materials and Methods

### Animals

128 male F344/Mol-rats, SPF (F344/Ntac@Mol) three to four weeks old were purchased from M & B (LI. Skensved, Denmark).

### Diets

All groups of rats are offered a powdered purified diet. The amounts of 1α,25(OH)₂D₃, calcium and acetylsalicylic acid in the diet for each group are presented in table 1.

### Chemical

Azoxymethane (AOM) was obtained from Sigma Chemical (St. Louis, MO). 1α,25(OH)₂D₃ was provided by Leo Pharmaceutical Products, Ballerup, Denmark. Acetylsalicylic acid (ASA) was obtained from Nycomed Danmark A/S.

### Housing

The animals were kept in disposable plastic cages with an inserted steel grid floor, two animals per cage, in flexible film isolaters (Isotec 12134, Olac, Oxford, UK) during the dosing period, and one week after the termination of the dosing with AOM. For the remaining period of the study, the animals were kept in stainless steel wire cages with two animals per cage. During the study, the temperature was maintained at 21 ± 1° C, a relative humidity at 55 ± 5%, air was changed 10 times/hr, and fluorescent light was on from 2100 to 0900.

### Experimental design

The animals were randomly assigned to eight experimental groups of 16 animals and fed their respective diets for 19 days (table 1). Then all groups were dosed AOM, 15 mg/kg body weight subcutaneously twice one week apart. The animals were maintained on their respective diets for a 16-week observation period. Body weight, food and water consumption were measured weekly. At termination of the study, ten animals from each of the groups 1, 6, 7, and 8 were placed separately in metabolism cages for 24 hours. After rinsing with 10 ml water the urine was collected for determination of volume, pH, calcium and creatinine. Eighteen weeks after the first AOM injection the animals were sacrificed and serum was collected for future analysis of acetylsalicylic acid. The abdomen and thoracic cavity of all animals were examined to reveal macroscopic changes. The weight of kidneys and adrenals were recorded. These organs, the stomach, small intestine, and thyroid gland with parathyroids were preserved in 4% buffered formaldehyde, pending future requirement for histopathological examination. The large intestine was cut longitudinally, rinsed in 0.9% NaCl, and divided into two pieces of equal length, pinned on a cork slab, and fixed in cold 4% neutral buffered formaldehyde according to (1).

**Table 1:**

| Scheme of treatment | | | | |
|---|---|---|---|---|
| ***Group*** | ***Animal no.*** | ***ASA*** ppm | 1α,25(OH)₂D₃ µg/ kg * | ***Ca*** Ppm |
| 1 | 1-16 | 0 | 0 | 5000 |
| 2 | 17-32 | 0 | 0 | 2500 |
| 3 | 33-48 | 0 | 0 | 7500 |
| 4 | 49-64 | 0 | 0.02 | 7500 |
| 5 | 65-80 | 300 | 0 | 7500 |
| 6 | 81-96 | 300 | 0.02 | 7500 |
| 7 | 97-112 | 300 | 0.02 | 5000 |
| 8 | 113-128 | 300 | 0.02 | 2500 |

| | | | | |
|---|---|---|---|---|
| *) The content of vitamin D₂ in SYN 8 is unchanged 1000 IU/ kg. | | | | |

### Urine analysis

Analysis for creatinine and calcium were analysed using a Combas Mira S analyser using the relevant kit for each parameter (Roche Diagnostic Systems).

### Assessment of ACF

The ACF were visualised by Giemsa staining, recorded using a stereomicroscope at x40 magnification, and grouped into small (1-3 crypts), medium (4-6 crypts), large (7-9 crypts), extra-large (≥ 10 crypts), and ACF>7 (> 7 crypts). The ACF were distinguished from normal crypts according to (1).

### Assessment of tumors

Colonic tissue deviations suspected to be tumors at gross examination or under stereomicroscope were embedded in paraffin, sectioned 4-6 µm, and stained with hematoxylin and eosin for histopathological examination.

### Statistical analysis

All data are presented as mean ± SE. One way analysis of variance with repeated measures on one factor was used to analyse body weight and food and water consumption. The analyses were followed by a Least Significant Difference test if significant. Data on organ weights, urine chemistry, and ACF was analysed by an one-way analysis of variance followed by a Least Squares Means. The homogeneity of variance among groups was evaluated by judgement of standard residual plots (General Linear Model procedure). Data on tumor bearing animals was analysed by Fishers exact test. A p < 0.05 was considered significant. All statistical analysis was carried out using SAS release 6.12.

### Results and discussion

### Urine chemistry

The urinary volume, pH, concentration of calcium and creatinine of group 1, 6, 7, and 8 were examined (see Table 2. The concentration of calcium was statistically significant decreased in group 6 and 8. A non-significant decrease was observed in group 7 as well. The effect is likely to be caused by a 1α,25(OH)₂D₃ induced renal retention of calcium. The pH of group 7 was statistically significant higher than the control value. As no significant changes were observed in groups 6 and 8 this is probably a fortuitousness. The results further demonstrate that no side effect of the combination treatment according to the invention is observed.

**Table 2.**

| Urine chemistry ^{a} | | | | | |
|---|---|---|---|---|---|
| Group ^{b} | N | volume ml | pH | calcium mmol/l | creatinine µmol/l |
| 1 | 10 | 11.3 ± 1 | 7.2 ± 1.1 | 6.57 ± 1.9 | 7062 ± 560 |
| 6 | 10 | 12.1 ± 2 | 6.9 ± 1.1 | 4.49 ± 1.6* | 7412 ± 858 |
| 7 | 10 | 11.8 ± 1 | 8.6 ± | 5.80 ± 2.0 | 7164 ± 429 |
| 8 | 10 | 12.0 ± 2 | 7.8 ± 1.1 | 2.8 ± 1.7* | 6370 ± 512 |

| | | | | | |
|---|---|---|---|---|---|
| a: Mean ± SD. | | | | | |
| b: Group 1, control diet (5000 ppm Ca); group 6, 0.02 µg 1α,25(OH)₂D₃, 300 ppm acetyl salicylic acid, and 7500 ppm Ca in the diet; group 7, 0.02 µg 1α,25(OH)₂D₃, 300 ppm acetyl salicylic acid, and 5000 ppm Ca in the diet; group 8, 0.02 µg 1α,25(OH)₂D₃, 300 ppm acetyl salicylic acid, and 2500 ppm Ca in the diet. | | | | | |
| * mark statistically significance to control group when a Least Square Means test was performed (p<0.05). | | | | | |

### Organ weight

The relative organ weight was calculated per 100g body weight. The terminal body weight, the absolute weight of kidneys and adrenals, and the relative weight of adrenals were not affected by treatment. The relative kidney weight of group 5 and 8 was increased when compared to the control group. The changes are minor and not believed to be related to treatment.

### ACF and Tumors

ACF as intermediary bio-markers and not tumors/cancers were end-points for this study. Nevertheless a number of animals developed tumors during the trial. According to the adenoma - carcinoma sequence tumors represent late-stage carcinogenesis and are therefore included in the results. Large ACF, x-large ACF and tumors represent lesions with significant correlation to subsequent invasive cancer and cancer related death. The group of animals with lesions containing more than seven crypts (ACF>7) is considered as high-risk animals with regard to cancer development.

The total number and distribution of ACF and tumors are presented in Table 3 and appendix 3.
Animals fed on a low calcium diet of 2500 ppm (mimicking an older ,western-world, human population) were most susceptible to ACF development. Increasing calcium levels in the diet significantly reduced the number of ACF. In animals fed 7500 ppm calcium and 1,25(OH)₂-D₃ a statistically significant decrease in the total number of ACF was observed when compared to group 1 and 2 (35.1 vs. 61.7, and 35.1 vs. 88.3, respectively). In animals fed 7500 ppm calcium, 1,25 (OH)₂D₃ and ASA a non-significant reduction in total ACF was observed when compared to group 1 (44.6 vs. 61.7).

**Table 3**

| Mean number of aberrant crypt foci (ACF) or tumors in AOM-induced rats fed diets containing 1α,25(OH)₂D₃, acetyl salicylic acid, and various doses of Calcium^{a-b} | | | | | | | | |
|---|---|---|---|---|---|---|---|---|
| Group^{c} | N | Total | Small | Medium | Large | Extra Large | Tumors | ACF>7 crypts |
| 1 | 15 | 61.7 ± 8.4 | 39.8 ± 5.0 | 18.9 ± 3.4 | 2.3 ± 0.7 | 0.7 ± 0.3 | 0.2 ± 0.1 | 3.2 ± 0.8 |
| 2 | 16 | 88.3 ± 9.4 | 55.1 ± 6.3 | 27.6 ± 3.8 | 4.5 ± 0.9 | 1.1 ± 0.4 | 0.6 ± 0.2 | 6.1 ± 1.3 |
| 3 | 16 | 46.8 ± 8.7 | 35.4 ± 6.5 | 9.9 ± 2.3 | 1.0 ± 0.3 | 0.4 ± 0.2 | 0.9 ± 0.3 | 2.4 ± 0.6 |
| 4 | 16 | 35.1 ± 5.0 | 26.1 ± 3.5 | 7.5 ± 1.5 | 1.1 ± 0.3 | 0.4 ± 0.1 | 1.1 ± 0.3 | 2.6 ± 0.5 |
| 5 | 16 | 45.4 ± 5.7 | 33.0 ± 5.1 | 10.9 ± 1.7 | 1.2 ± 0.4 | 0.3 ± 0.1 | 0.5 ± 0.2 | 1.9 ± 0.6 |
| 6 | 16 | 44.6 ± 6.7 | 32.9 ± 5.0 | 10.4 ± 2.1 | 0.6 ± 0.3 | 0.6 ± 0.3 | 0.5 ± 0.2 | 1.8 ± 0.6 |
| 7 | 16 | 73.6 ± 8.8 | 52.2 ± 6.0 | 19.3 ± 2.9 | 1.8 ± 0.3 | 0.3 ± 0.1 | 0.8 ± 0.3 | 2.9 ± 0.7 |
| 8 | 16 | 90.3 ± | 63.7 ± 7.0 | 23.3 ± 3.4 | 3.1 ± 0.9 | 0.3 ± 0.1 | 0.1 ± 0.1 | 3.4 ± 0.9 |

| | | | | | | | | |
|---|---|---|---|---|---|---|---|---|
| a: Mean ± SE | | | | | | | | |
| b: Small foci: 1-3 crypts; Medium foci: 4-6 crypts; Large foci: 7-9 crypts; Extra large foci: ≥ 10 crypts; ACF>7: >7 crypts | | | | | | | | |
| c: Group 1: Normal Calcium control group (5000 ppm Ca); Group 2: Low Calcium control group (2500 ppm Ca); Group 3: Supplemental Calcium group (7500 ppm Ca); Group 4: 0.02 µg 1α,25(OH)₂D₃ and 7500 ppm Ca; Group 5: 300 ppm acetyl salicylic acid and 7500 ppm Ca; Group 6: 0.02 µg 1α,25(OH)₂D₃, 300 ppm acetyl salicylic acid, and 7500 ppm Ca; Group 7: 0.02 µg 1α,25(OH)₂D₃, 300 ppm acetyl salicylic acid, and 5000 ppm Ca; Group 8: 0.02 µg 1α,25(OH)₂D₃, 300 ppm acetyl salicylic acid, and 2500 ppm Ca | | | | | | | | |

When comparing the high-risk group (ACF>7) on the low calcium diet (2500 ppm) to the high-risk group treated with the three active components (group 6), a highly significant reduction in the number of colonic lesions (ACF>7) (6.1 vs. 1.8, p=0.0001) is seen.

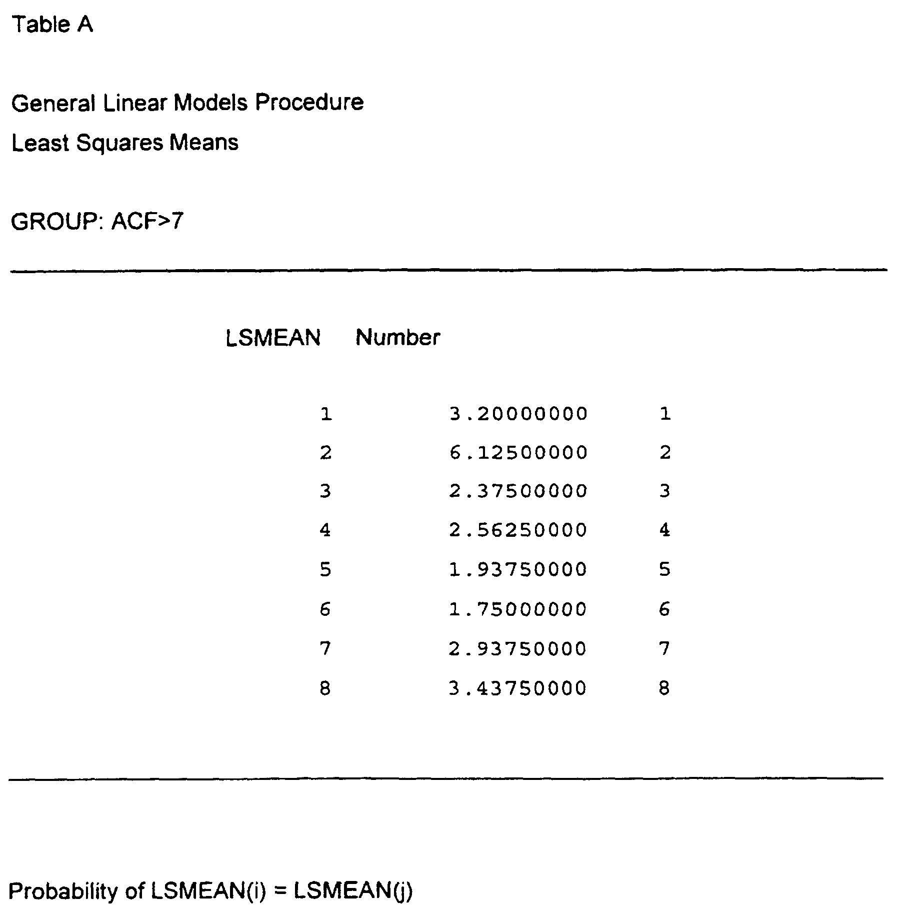

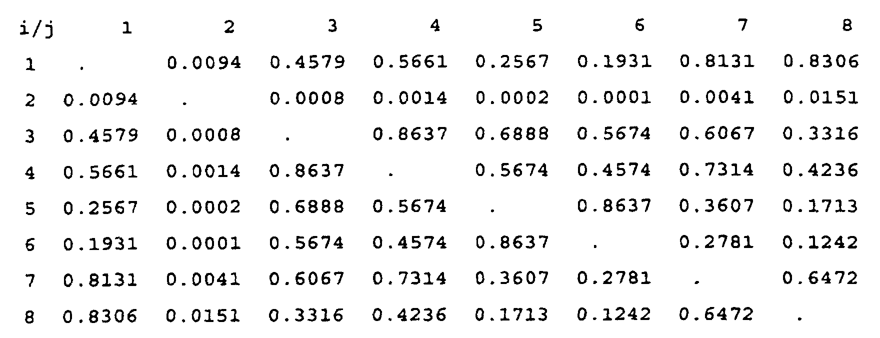

Separate addition of Calcium, 1α,25 (OH)₂D₃ or ASA, or combinations of two, also gave significant reductions in ACF indicating a protective effect. The most pronounced effect was obtained with the combination of all 3 ingredients, suggesting an additive/synergistic effect.
When regarding low calcium diets, the increase in total number of ACF in group 8 was primarily due to an increase in small ACF. Whereas in group 2, in addition to a non statistically significant increase in small ACF (p=0.061), statistically significant increases in medium, large sized ACF and ACF>7 were observed. This demonstrates that addition of 1α,25(OH)₂D₃ and ASA to the fodder protects against progression of small ACF in animals given sub-optimal calcium (2500 ppm). When increasing calcium to a higher level (5000 ppm), an obvious trend is seen (1.8 vs. 3.2, p=0.19) supporting the anticancer effect of 1α,25(OH)₂D₃ and ASA is recognised.

In conclusion, treatment of animals given a low calcium diet with the combination of 1α,25(OH)₂D₃, Ca, and ASA results in a statistically significant reduction in the development of pre-neoplastic colonic lesions.

### Tumors

Tumor bearing rats were observed in all groups (Table 4). The presented results should be taken with caution as further histiological examination is needed for final evaluation. At autopsy, some of the lesions appeared very prominent and tumor-like, reddish, elevated and one to fifthteen millimetre in diameter. Whereas others lesions were less elevated and had the same colour as the mucosa. Some animals had more than one tumor often 2 to 3. Other lesions were only observed when the intestine was investigated under stereomicroscope for counting of ACF. Tumors were also observed in the small intestine of one animal of group 1, 5, 7, and 8. In group 2, two animals carried tumors in their small intestine (not histiologically examined). The highest number of tumor bearing animals and total number of tumors were seen in group 3 and 4. Group 8 receiving the lowest level of calcium in combination with ASA and 1α,25(OH)₂D₃ had the lowest tumor burden, followed by the group 1. When comparing the numbers of tumor bearing animals of group 2 and 8 (both receiving calcium at the lowest level), the results indicate a protective effect of the ASA and 1α,25(OH)₂D₃ combination. A similar but less pronounced tendency is seen when comparing group 3 and 6 (both receiving the highest level of calcium).

Tumor data indicate that the combination of 1α,25(OH)₂D₃ and ASA given to animals fed either the high or low level of calcium (group 6 and 8) tended to reduce the number of tumor bearing animals and the total number of tumors when compared to the relevant control group receiving the same level of calcium. Although the reduction is non-statistically significant, this indicates protection against progression of ACF to adenomas. Especially at the low calcium level, where the highest total number of ACF was recorded but the lowest number of tumor bearing animals.

**Table 4.**

| Colo-rectal tumor in AOM-induced rats fed diets containing 1α,25(OH)₂D₃, acetyl salicylic acid, and various doses of Calcium - **preliminary data**^{**a**} | | | | |
|---|---|---|---|---|
| Group | N | No. of tumor bearing rats | No. of rats with adenoma | No. of rats wi adenocarcino |
| 1 | 15 | 2 | 1 | 2 |
| 2 | 16 | 6 | 4 | 4^{b} |
| 3 | 16 | 9 | 8 | 3 |
| 4 | 16 | 9 | 7 | 4 |
| 5 | 16 | 6 | 4 | 3 |
| 6 | 16 | 6 | 5 | 3 |
| 7 | 16 | 8 | 6 | 4^{b} |
| 8 | 16 | 1 | 0 | 1^{b} |

| | | | | |
|---|---|---|---|---|
| a: Group 1, control diet (5000 ppm Ca); group 2, 2500 ppm Ca in the diet; group 3, 7500 ppm Ca in the diet; group 4, 0.02 µg 1α,25(OH)₂D₃ in the diet; group 5, 300 ppm acetyl salicylic acid and 7500 ppm Ca in the diet, group 6, 0.02 µg 1α,25(OH)₂D₃, 300 ppm acetyl salicylic acid, and 7500 ppm Ca in the diet; group 7, 0.02 µg 1α,25(OH)₂D₃, 300 ppm acetyl salicylic acid, and 5000 ppm Ca in the diet; group 8, 0.02 µg 1α,25(OH)₂D₃, 300 ppm acetyl salicylic acid, and 2500 ppm Ca in the diet. | | | | |
| b: including mucinous adenocarcinoma and signet-ring carcinoma. | | | | |

### Example 2

The *in vivo* study of the effect on prevention of CRC in AOM (Azoxymethane) induced rats

The rats are induced s.c 1 x week in 2 weeks with an AOM solution.

The effect of the specific treatment is evaluated due to the number of aberrant crypt foci (ACF) of the colon/rectum in the AOM-induced rats. The aberrant crypt score is evaluated by stereomicroscopia (40 X) upon staining with Giemsa-solution (6 ml cone. Giemsa in 50 ml PBS, pH 7.1). The crypt size is categorised as small, medium, large and X-large. The number of animals is 80 with 16 in each group. The medicaments are administered with the food. The section is 18 weeks from the first dosage. The Ca in the food is 5000/10000 ppm, the 1,24 DHC is 2.5 microg/kg. Data for weight, food and water are registeres and analyzed

### Treatment:

- Group 1.: Control animals
- Group 2.: 200 ppm aspirin (acetyl salicylic acid)
- Group 3.: Ca and 1,25 DHC
- Group 4.: 200 ppm aspirin, Ca and 1,25 DHC
- Group 5.: 100 ppm aspirin, Ca and 1,25 DHC

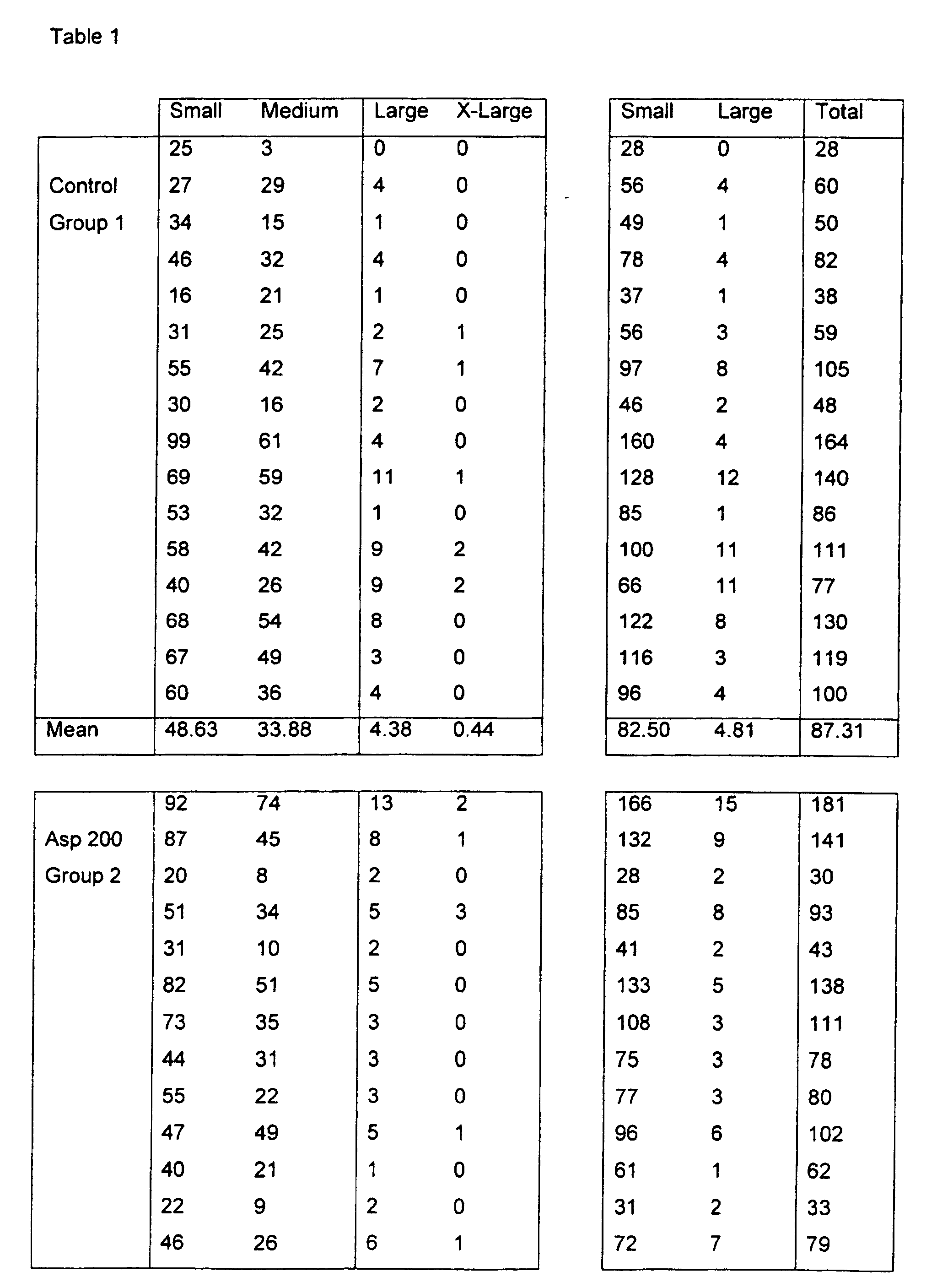

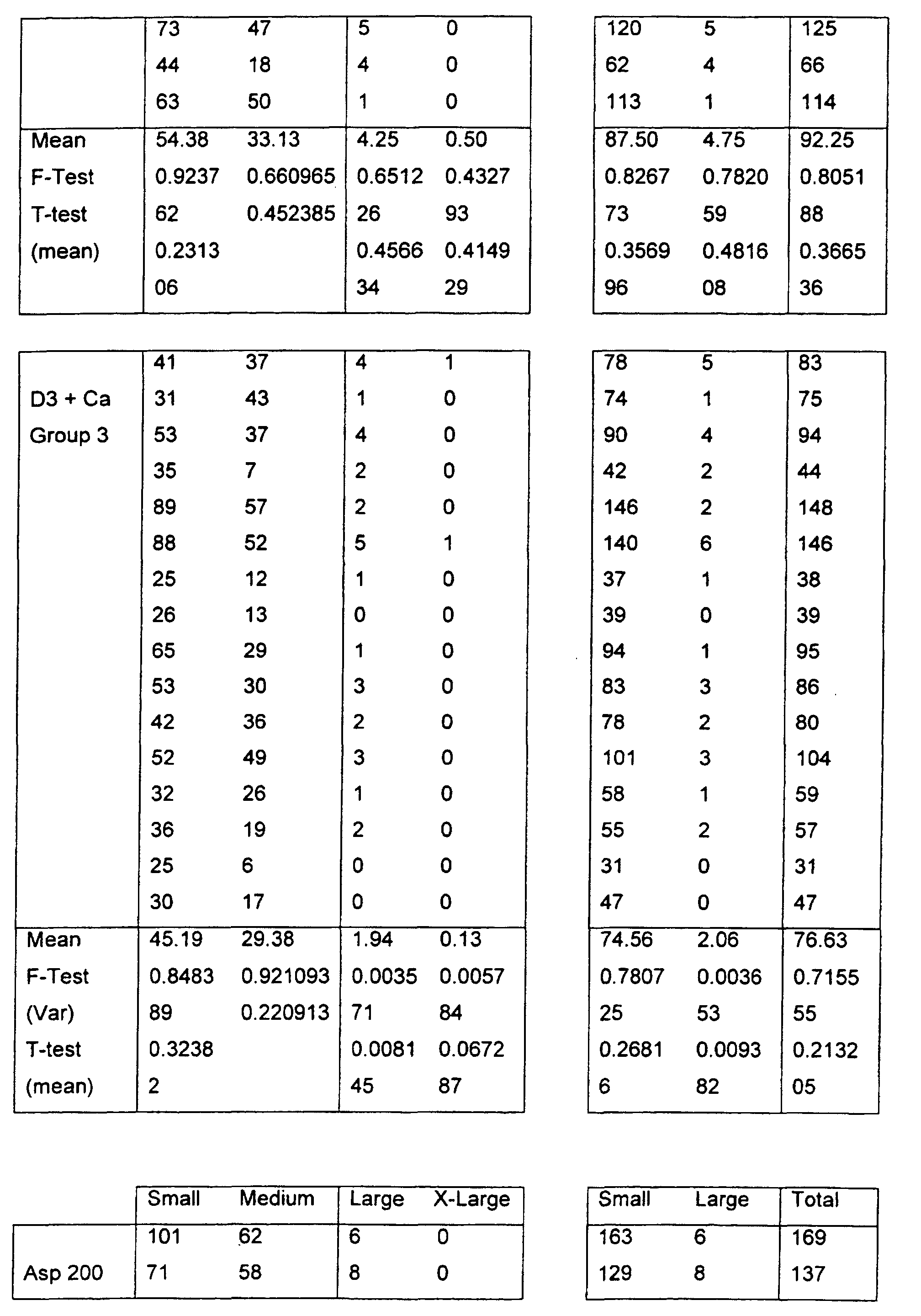

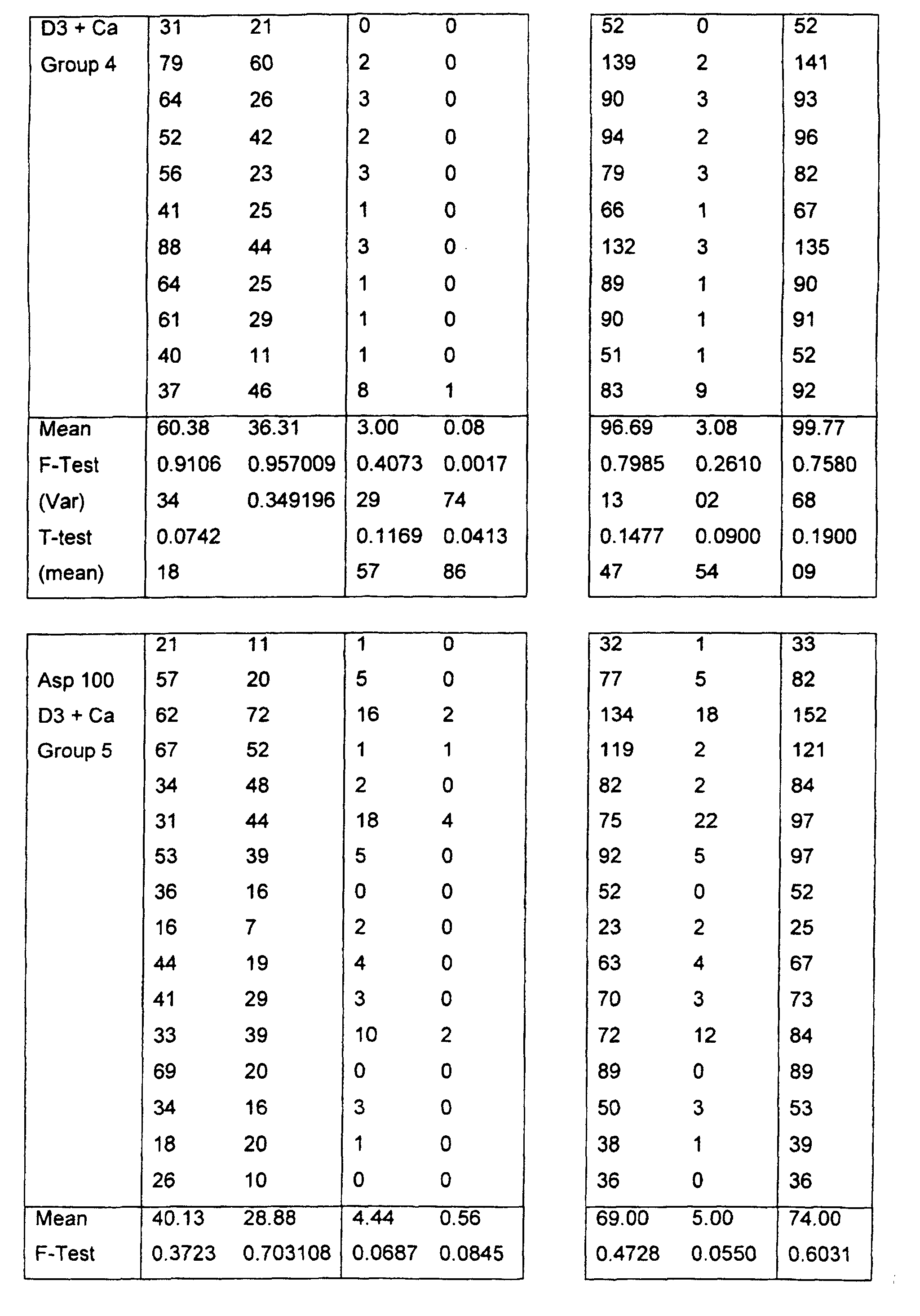

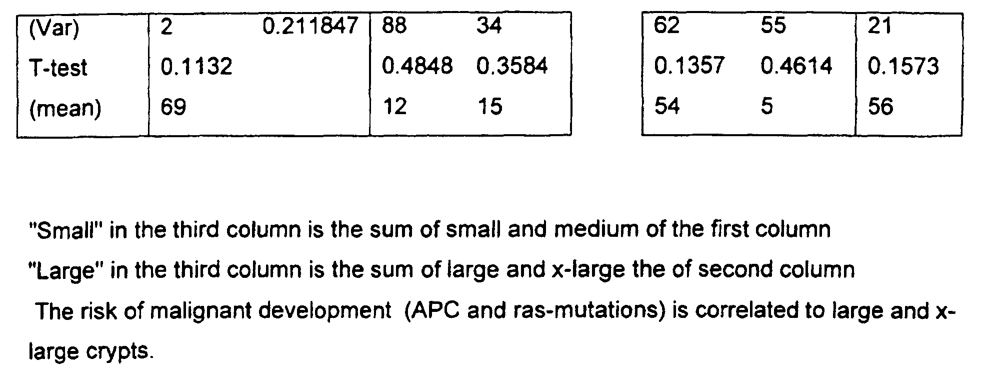

The aspirin dosage general considered as being preventive is 400-500 ppm in AOM-induced F344 rats.

The results shows that it is not possible to reduce the aspirin dosage to 200 ppm as this dosage is without effect (Group 2). However, the addition of Ca and 1,25 DHC results a significant reduction in large ACF (Group 4). The results of Group 3 and 4 are peculiar as it seems as though the presence of aspirin is an adverse effect. It should be noted that the rats suffered from hypercalcaemia as shown by kidney stones.

### References:

1. Trujillo MA, Garewal, HS, Sampliner RE. Non-steroidal anti-inflammatory agents in chemoprevention of colorectal cancer. At what cost?
   Dig Dis Sci 1994;39:2260-6.
2. Caplan LS, Hutton M, Muller DS & al. Secondary prevention of cancer.
   Curr Opin Oncol 1996;8:441-6.
3. Clemmesen IH, Storm H. Kræft in Danmark. En opslagsbog.
   Kræftens Bekæmpelse 1993.
4. Key JAK, Thorogood M, Appleby PN & al. Dietary habits and mortality in 11000 vegetarians and health conscious people: results of a 17 year follow up.
   BMJ 1996;313:7060.
5. Raskov H. Adjuverende systemisk kemoterapi ved cancer coli.
   Ugeskrift for Læger 1996;158:1222-7.
6. Garland CF, Barrett-Connor E, Ressof AH & al. Dietary vitamin D and calcium and risk of colorectal cancer: a 19-year prospective study in men.
   Lancet 1985;i:307-9.
7. Garland CF, Garland FC, Shaw EK & al. Serum 1,25 hydroxyvitamin D and colon cancer: eight-year prospective study.
   Lancet 1989;nov 18:1176-8.
8. Garland CF, Garland FC, Gorham ED. Can colon cancer incidence and death rates be reduced with calcium and vitamin D?
   Am J Clin Nutr 1991;54:193S-201S.
9. Kune GA, Kune S, Watson LF. Colorectal cancer risk, chronic illnesses, operations and medications: case control results from the Melbourne Colorectal Cancer Study.
   Cancer Res 1988;48:4399-404.
10. Suh O, Mettlin C, Petrelli NJ. Aspirin use, cancer and polyps of the large bowel.
   Cancer 1993;72:1171-7.
11. Thun MJ, Namboodiri MM, Heath CW Jr. Aspirin use and reduced risk of fatal colon cancer.
   N Engl J Med 1991;325:1593-6.
12. Buring JE, Lee IM, Hennekens CH. Non-steroidal inflammatory drugs and colorectal cancer.
   Cancer 1994;74:1837-9.
13. Heath CW, Thun MJ, Greenberg ER & al. NSAID and human cancer.
   Cancer 1994;74:2885-8.
14. Pence BC. Role of calcium in colon cancer prevention: experimental and clinical studies.
   Mut Res 1993;290:87-95.
15. Duris I, Hruby D, Pekarkova B, Huorka M, Cernakova E, Bezayova T & al. Calcium chemoprevention in colorectal cancer.
   Hepatogastroenterol 1996;43:152-4.
16. Buset M, Winaver MLS, Swaroop S, Friedmann E. Inhibition of human colonic epithelial cell proliferation in vivo and in vitro by calcium.
   Cancer Res 1986;46:5426-30.
17. Wargovich MJ, Lointier PH. Calcium and vitamin D modulate mouse colon epithelial proliferation and growth characteristics of a human colon tumor cell line.
   Can J Physiol Pharmacol 1987;65:472-7
18. IARC Handbooks of Cancer Prevention, vol 1. Non-steroidal anti-inflammatory drugs. Lyons: International Agency for Research on Cancer.
   In Press.
19. Dorudi S, Hanby AM, Poulsom R, Northover J, Hart IR. Levels of expression of E-cadherin m-RNA in colorectal cancer correlates with clinical outcome.
   Br J Cancer 1995;71:614-6.
20. Newmark HL, Lipkin M. Calcium, vitamin D and colon cancer.
   Cancer Research 1992;52:2067S-70S.
21. Rasmussen H. The calcium messenger system.
   N Engl J Med 1986;314:1094-1101.
22. Llor X, Jacoby RF, Teng BB, Davidson NO, Sitrin MD, Brasitus TA. K-ras mutations in 1,2 dimethylhydrazine induced colonic tumors: effects of supplemental dietary calcium and vitamin D deficiency.
   Cancer Res 1991;51:4305-9.
23. Reshef R, Rozen P, Fereman Z, Fine N, Barzilai M, Sasha SM, Shkolnik T. Effect of a calcium enriched diet on the colonic epithelium hyperproliferation induced by N-methyl-N-nitro-N-nitrosoguanidine in rats on a low calcium and fat diet.
   Cancer Res 1990;50:1764-7.
24. Kane KF, Mitchell NP, Langman MJS & al. Functional vitamin D₃ receptors are present in human colorectal neoplasms.
   Gastroenterology 1995; 108:A487.
25. Anticancer Res 1996;16:2333-8.
26. Eisman JA, Barkla DH, Tutton PJM. Suppression of in vivo growth of human cancer solid xenografts by 1,25-dihydroxyvitamin D₃.
   Cancer Res 1987;47:21-5.
27. Newmark HL, Lipkin M. Calcium, Vitamin D and Colon Cancer.
   Cancer Res 1992;52s:2067s-70s.
28. Newmark HL, Lipkin M. Calcium, Vitamin D and Colon Cancer.
   Cancer Res 1992;52s:2067s-70s.
29. Ternent C, Ding XZ, Adrian T. Lipoxygenase blockade inhibits growth factor-induced colonic cancer cell proliferation.
   Abstract, ASCRS Ann Meeting 1999, Washington DC.
30. Marcus AJ. Aspirin as prophylaxis against colorectal cancer.
   N Engl J Med 1995;333:656-8.
31. Frolich JC. A classification of NSAIDs according to the relative inhibition of cyclooxygenase enzymes.
   TIPS 1997;18:30-4.
32. Hanif AP, Feng Y, Koutsos M & al. NSAIDs inhibit the growth of colon cancer cell lines by a prostaglandin independent pathway.
   Gastroenterology 1995;108:A478.
33. Ahnen D, Piazza G, Alberts D & al. Sulindac sulfide and sulfone both inhibit the growth of colon cancer cell lines by inducing apoptosis.
   Gastroenterology 1995;108:A443.
34. Alberts DS, Hixson LJ, Ahnen D, Bogert C, Einspahr J, Paranka N & al.
   Do NSAIDs exert their colon cancer chemoprevention activities through the inhibition of mucosal prostaglandin synthetase?
   J Cell Biochem 1995;s22:18-23.
35. Kahlenberg M, Stoler D, Volpe C, Petrelli N, Anderson G. Nonsteroidal anti-inflammatory drugs (NSAID's) reduce genomic instability in colorectal tumor cells.
   Abstract. Surgical Oncology Societies 51st annual cancer symposium 1998.
36. Levy G. Prostaglandin H synthases, nonsteroidal anti-inflammatory drugs and colon cancer.
   FASEB J 1997;11:234-47.
37. Hixson LJ, Alberts DS, Krutzsch M, Einspahr J, Brendel K, Gross PH & al. Antiproliferative effect of non-steroidal anti-inflammatory drugs against human colon cancer cells.
   Cancer Epidemiol Biomarkers Prev 1994;3:5 433-8.
38. Dubois RN, Giardello FM, Smalley WE. Nonsteroidal anti-inflammatory drugs, eicosanoids and colorectal cancer prevention.
   Gastroenterol Clin N Am 1996;25:773-91.
39. Watson AJ. Chemopreventive effects of NSAIDs against colorectal cancer: regulation of apoptosis and mitosis by COX-1 and COX-2.
   Histol Histopathol 1998;13:591-7.
40. Steering Committee of the Physicians' Health Study Research Group. Final report on the aspirin component of the ongoing Physician Health Study.
   N Engl J Med 1989;321:129-35.
41. Muscat JE, Stellman SD, Wynder EL. NSAID and colorectal cancer
   Cancer 1994;74:1847-54.
42. Giovannucci E, Egan KM, Hunter DJ & al. Aspirin and the risk of colorectal cancer.
   N Engl J Med 1995;333:609-14.
43. Giovannucci E, Rimm EB, Stampfer MJ & al. Aspirin use and the risk of colorectal cancer and adenoma in male health professionals.
   Ann Int Med 1994;121:241-6.
44. Pollard M, Luckert PH. Effect of indomethacin on intestinal tumors induced in rats by the acetate derivative of dimethylnitrosamine.
   Science 1981;214:558-9.
45. Narisawa T, Sato M, Tani M & al. Inhibition of development of methylnitrosurea induced rat colon tumors by indomethacin treatment.
   Cancer Res 1981;41:1954-7.
46. Reddy BS, Maruyama H, Kelloff G. Dose-related inhibition of colon carcinogenesis by dietary piroxicam, a nonsteroidal anti-inflammatory drug, during different stages of rat colon tumor development.
   Cancer Res 1987;47:5340-6.
47. Kudo T, Narisawa T, Abo S. Antitumor activity of indomethacin on methylazoxymethanol-induced large bowel tumors in rats.
   Gann;71:260-4.
48. CAPRIE Steering Commitee. A randomised, blinded, trial of clopidogrel versus aspirin in patients at risk of aschaemic events (CAPRIE).
   Lancet 1996;348:1329-39.
49. Duffy MA, (ed.), Physicians' Desk Reference. Montvale, NJ.
   Medical Economics Data, p. 780, 1993.
50. McEvoy GK, McQuarrie GM. Aspirin. In: Drug Information 86, Bethesda, MD: American Society of Hospital Pharmacists 1986:841-8.
51. Kristiansen, E, Thorup, I, and Meyer, O: Influence of different diets on development of DMH-induced aberrant crypt foci and colon tumor incidence in Wistar rats. *Nutr Cancer* 23, 151-159, 1995.
52. Diaz, Dario, et al.: Apoptosis is induced by the active metabolite of vitamin D₃ and its analogue EB1089 in colorectal adenoma and carcinoma cells: possible implications for prevention and therapy: Cancer research 60, 2304-2312, 2000.
53. Dwerryhouse, D. J., et al.: 'Non-cytotoxic control of colorectal cancer': J.R. Coll.Surg. Edinb., Volume 42, June 1997, pages 147 - 153.
54. Pence, Barbara C., et al.: 'Chemopreventive effects of calcium but not aspirin supplementation in cholic acid-promoted colon carcinogenesis : correlation with intermediate endpoints ': Carcinogenesis, Volume 16, No. 4, 1995, pages 757 - 765.

## Claims

1. A pharmaceutical medicament comprising a combination of acetylsalicyclic acid (ASA), 1,25 dihydrocholecalciferol (1,25 DHC) and calcium in a combination dosage together with a pharmaceutically acceptable carrier.

2. A pharmaceutical medicament according to claim 1 wherein the combination dosage comprises 1,25 DHC in the range of 0,1 µg to 2 µg.

3. A pharmaceutical medicament according to claim 1 or 2 wherein the combination dosage comprises calcium in the range of 200 mg to 3000 mg.

4. A pharmaceutical medicament according to any of claims 1 to 3 wherein the combination dosage comprises ASA in the range of 50 mg to 500 mg.

5. A pharmaceutical medicament according to any of claims 1 to 4 wherein the combination dosage comprises 50 to 75 mg ASA, 500 to 1000 mg calcium, and 0,5 µg to 1 µg 1,25 DHC.

6. Use of acetylsalicylic acid (ASA), the vitamin D₃ metabolite 1,25 dihydroxycholecalciferol (1,25 DHC) and calcium together with a pharmaceutically acceptable carrier for the preparation of a medicament for preventing the initiation and/or progression of colorectal cancer in a human.

7. Use according to claim 6 wherein the acetylsalicylic acid (ASA) is in the range of 50 mg to 500 mg.

8. Use according to claim 6 or 7 wherein calcium is in the range of 200 mg to 3000 mg.

9. Use according to any of claims 6-8 wherein 1,25 DHC is present in an amount of 0,1 µg to 2 µg.

10. Use according to any of claims 6-9 wherein the combination is in the form of a combination dosage comprising ASA, 1,25 DHC and calcium.

## Patentansprüche

1. Pharmazeutisches Medikament umfassend eine Kombination aus Acetylsalicylsäure (ASA), 1,25-Dihydrocholecalciferol (1,25-DHC) und Calcium in einer Kombinationsdosierung zusammen mit einem pharmazeutisch annehmbaren Träger.

2. Pharmazeutisches Medikament nach Anspruch 1, worin die Kombinationsdosierung 1,25-DHC im Bereich von 0,1 µg bis 2 µg umfasst.

3. Pharmazeutisches Medikament nach Anspruch 1 oder 2, worin die Kombinationsdosierung Calcium im Bereich von 200 mg bis 3000 mg umfasst.

4. Pharmazeutisches Medikament nach den Ansprüchen 1 bis 3, worin die Kombinationsdosierung ASA im Bereich von 50 mg bis 500 mg umfasst.

5. Pharmazeutisches Medikament nach den Ansprüchen 1 bis 4, worin die Kombinationsdosierung 50 bis 75 mg ASA, 500 bis 1000 mg Calcium und 0,5 µg bis 1 µg 1,25-DHC umfasst.

6. Verwendung von Acetylsalicylsäure (ASA), dem Vitamin-D₃-Metaboliten 1,25-Dihydroxycholecalciferol (1,25-DHC) und Calcium zusammen mit einem pharmazeutisch annehmbaren Träger für die Herstellung eines Medikaments zur Prävention der Entstehung und/oder Progression von kolorektalem Krebs beim Menschen.

7. Verwendung nach Anspruch 6, worin die Acetylsalicylsäure (ASA) im Bereich von 50 mg bis 500 mg liegt.

8. Verwendung nach Anspruch 6 oder 7, worin Calcium im Bereich von 200 mg bis 3000 mg liegt.

9. Verwendung nach den Ansprüchen 6 bis 8, worin 1,25-DHC in einer Menge von 0,1 µg bis 2 µg vorliegt.

10. Verwendung nach den Ansprüchen 6 bis 9, worin die Kombination in Form einer Kombinationsdosierung umfassend ASA, 1,25-DHC und Calcium vorliegt.

## Revendications

1. Médicament pharmaceutique comprenant une combinaison d'acide acétylsalicylique (ASA), de 1,25-dihydrocholécaldiférol (1,25 DHC) et de calcium sous combinaison dosée avec un véhicule pharmaceutiquement acceptable.

2. Médicament pharmaceutique selon la revendication 1 dans lequel la combinaison dosée comprend du 1,25 DHC dans l'intervalle de 0,1 µg à 2 µg.

3. Médicament pharmaceutique selon la revendication 1 ou 2 dans lequel la combinaison dosée comprend du calcium dans l'intervalle de 200 mg à 3 000 mg.

4. Médicament pharmaceutique selon l'une quelconque des revendications 1 à 3 dans lequel la combinaison dosée comprend de l'ASA dans l'intervalle de 50 mg à 500 mg.

5. Médicament pharmaceutique selon l'une quelconque des revendications 1 à 4 dans lequel la combinaison dosée comprend 50 à 75 mg d'ASA, 500 à 1 000 mg de calcium et 0,5 µg à 1 µg de 1,25 DHC.

6. Utilisation de l'acide acétylsalicylique (ASA), du métabolite de la vitamine D₃ 1,25-dihydroxycholécalciférol (1,25 DHC) et de calcium ensemble avec un véhicule pharmaceutiquement acceptable pour la préparation d'un médicament pour prévenir l'initiation et/ou la progression du cancer colorectal chez un humain.

7. Utilisation selon la revendication 6 dans laquelle l'acide acétylsalicylique (ASA) est dans l'intervalle de 50 mg à 500 mg.

8. Utilisation selon la revendication 6 ou 7 dans laquelle le calcium est dans l'intervalle de 200 mg à 3 000 mg.

9. Utilisation selon l'une quelconque des revendications 6 à 8 dans laquelle la 1,25 DHC est présente en une quantité de 0,1 µg à 2 µg.

10. Utilisation selon l'une quelconque des revendications 6 à 9 dans laquelle la combinaison est sous la forme d'une combinaison dosée comprenant de l'ASA, du 1,25 DHC et du calcium.
